(19) Europäisches Patentamt
European Patent Office
Office européen des brevets

(11) **EP 4 520 282 A1**

(12) **EUROPEAN PATENT APPLICATION**
published in accordance with Art. 153(4) EPC

(43) Date of publication:
**12.03.2025 Bulletin 2025/11**

(21) Application number: **22940755.6**

(22) Date of filing: **28.10.2022**

(51) International Patent Classification (IPC):
**A61B 18/14** (2006.01)   **G06F 17/14** (2006.01)
**G16H 40/63** (2018.01)

(86) International application number:
**PCT/CN2022/128236**

(87) International publication number:
**WO 2023/213061 (09.11.2023 Gazette 2023/45)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:   **05.05.2022   CN 202210481725**

(71) Applicant: **Innolcon Medical Technology (Suzhou)
Co., Ltd.
Jiangsu 215000 (CN)**

(72) Inventors:
 • **YAO, Longyang
  Suzhou, Jiangsu 215000 (CN)**

 • **ZHANG, Haoqi
  Suzhou, Jiangsu 215000 (CN)**
 • **WANG, Fuyuan
  Suzhou, Jiangsu 215000 (CN)**
 • **WEI, Dalun
  Suzhou, Jiangsu 215000 (CN)**
 • **LIU, Zhenzhong
  Suzhou, Jiangsu 215000 (CN)**
 • **LUO, Wei
  Suzhou, Jiangsu 215000 (CN)**

(74) Representative: **AWA Sweden AB
Matrosgatan 1
Box 5117
200 71 Malmö (SE)**

(54) **ELECTROSURGICAL GENERATOR, ELECTROSURGICAL SYSTEM, AND CONTROL METHOD THEREFOR**

(57)   The embodiment of the present application provides an electrosurgical generator, an electrosurgical system and a control method thereof. The method includes: executing a plurality of sub-processes sequentially after it is determined that tissue has been effectively clamped between two electrodes of a cutter; each sub-process includes a state determination stage and a tissue fusion stage; wherein in the state determination stage of each sub-process, at least one control parameter and at least one ending parameter of the current sub-process is determined based on at least one impedance parameter of the tissue and at least one time parameter; in the tissue fusion stage of each sub-process, energy is output to the tissue according to the at least one control parameter of the current sub-process, and it is determined whether the current sub-process should be ended according to the at least one ending parameter of the current sub-process. Therefore, the technical solution provided by the embodiment of the present application can adaptively control the process of tissue closure, dynamically and accurately control the energy output to the tissue according to the closure state of the tissue, and improve the success rate of tissue closure and the bursting pressure of the tissue.

EP 4 520 282 A1

**(Cont. next page)**

FIG.5

## Description

### Technical Field

The present application is related to the technical field of surgical instruments, and in particular to an electrosurgical generator, an electrosurgical system and a control method thereof.

### Background

An electrosurgical system (e.g., a high-frequency electrosurgical cutter system) includes an electrosurgical generator and a cutter connected thereto. When used in monopolar surgery, the electrosurgical system may also include a return electrode. The electrosurgical generator applies energy to a target tissue site via the cutter to achieve electrocutting, electrocoagulation, or tissue closure. In monopolar surgery, high-frequency current is applied to the target tissue site via a monopolar cutter, and then returns to the generator through the human body and a return electrode attached to a surface of the human body. In bipolar surgery, high-frequency current returns to the electrosurgical generator via the two electrodes of the cutter and the tissue clamped between the two electrodes. In bipolar surgery, since the current is confined between the two electrodes and does not flow through the surface of the human body, a safer and more reliable operation may be realized.

Tissue closure refers to a process of fusing collagen elastin and matrix on both sides of the tissue via the applied energy, so that they may form a fusion mass volume without obvious boundaries. The high-frequency electrosurgical cutter system working in bipolar mode may output high-frequency electrical signals to the tissue to fuse the tissue via electrical energy and realize tissue closure. However, the current high-frequency electrosurgical cutter system lacks the ability to reasonably output energy in real time according to the state of the tissue, which makes it easy for tissue adhesion and carbonization to occur during tissue closure, reducing success rate of tissue closure, and degrading bursting pressure of the tissue.

### Summary

The embodiments of the present application provide an electrosurgical generator, an electrosurgical system and a control method thereof, which can adaptively control the process of tissue closure, dynamically and accurately control the energy output to the tissue according to the closure state of the tissue, and improve the success rate of tissue closure and increases the bursting pressure of the tissue.

According to a first aspect, an embodiment of the present application provides an electrosurgical generator including: a power output module configured to output energy to a tissue via two electrodes of a cutter; and a control module that is configured to: determine at least one impedance parameter of the tissue based on sampling signals of the output energy; and execute a plurality of sub-processes sequentially after it is determined that the tissue is effectively clamped between the two electrodes of the cutter; wherein during each sub-process, at least one control parameter and at least one ending parameter of the current sub-process is determined based on the at least one impedance parameter of the tissue and at least one time parameter; and the power output module is controlled to output energy to the tissue according to the at least one control parameter of the current sub-process, and it is determined whether the current sub-process should be ended according to the at least one ending parameter of the current sub-process.

Optionally, the control module is configured to: in each sub-process, determine at least one control parameter of current sub-process according to a minimum impedance of the tissue in a previous sub-process and a duration of the previous sub-process.

Optionally, the control module is configured to: in each sub-process, determine an ending impedance of current sub-process according to a minimum impedance and a bias impedance of the tissue in a previous sub-process; wherein the bias impedance is a fixed value, or a value that changes with sub-processes.

Optionally, the control module is configured to end the current sub-process if a real-time impedance of the tissue is greater than the ending impedance of the current sub-process.

Optionally, the control module is configured to end the current sub-process if a duration of outputting energy to the tissue according to the at least one control parameter of the current sub-process is greater than a preset timeout period.

Optionally, the control module is configured to end the current sub-process if a duration of the current sub-process is greater than a preset sub-process maximum duration.

Optionally, the control module is further configured to: in each sub-process, determine an ending impedance at tissue closure according to a minimum impedance of the tissue in a previous sub-process, a minimum impedance of the tissue in all sub-processes before current sub-process, and an initial impedance of the tissue.

Optionally, the control module is further configured to: before outputting energy to the tissue according to the at least one control parameter of current sub-process, determine whether tissue closure is completed based on a real-time impedance of the tissue and the ending impedance at tissue closure, wherein it is determined that tissue closure is completed when a real-time impedance of the tissue is greater than the ending impedance at tissue closure.

Optionally, the control module is further configured to: in determination of ending the current sub-process, determine whether tissue closure operation has timed out; and proceed to the next sub-process when it is determined that the tissue closure operation is not timed out.

Optionally, the control module is further configured to: obtain an initial impedance and initial phase of the tissue, where the initial phase of the tissue is an initial value of a phase difference between the voltage and current output to the tissue by the two electrodes of the cutter; and determine whether the tissue is effectively clamped by the two electrodes of the cutter based on the initial impedance and the initial phase.

Optionally, the control module is further configured to: according to the initial phase, look up a table to obtain an impedance range corresponding to the initial impedance; determine whether the initial impedance is within its corresponding impedance range; if yes, it is determined that the tissue is effectively clamped by the two electrodes of the cutter; if not, it is determine that the tissue is not effectively clamped by the two electrodes of the cutter.

Optionally, the control module is further configured to: calculate an effective voltage value and an effective current value of the output energy based on the sampling signals, and calculate a reference impedance of the tissue according to the effective voltage value and effective current value; calculate a voltage peak value and a current peak value for a base frequency of the output energy, as well as a voltage peak value and a current peak value for the second harmonic based on the sampling signals using a discrete Fourier transform algorithm; calculate a base frequency impedance according to the voltage peak value and current peak value of the base frequency, and calculate a second harmonic impedance according to the voltage peak value and current peak value of the second harmonic; determine a weight coefficient of the base frequency impedance based on a ratio of the reference impedance to the base frequency impedance, and determine a weight coefficient of the second harmonic impedance based on a ratio of the reference impedance to the second harmonic impedance; and weighted average the base frequency impedance and the second harmonic impedance based on the weight coefficient of the base frequency impedance and the weight coefficient of the second harmonic impedance, to obtain a real-time impedance of the tissue.

Optionally, the control module is further configured to: calculate a reference phase of the output energy based on a voltage zero-crossing time point and a current zero-crossing time point in the sampling signals; calculate a voltage phase and a current phase of the base frequency of the output energy, as well as a voltage phase and a current phase of the second harmonic of the output energy, using a discrete Fourier transform algorithm based on the sampling signals; calculate a base frequency phase based on the voltage phase and current phase of the base frequency, and calculate a second harmonic phase based on the voltage phase and current phase of the second harmonic; determine a weight coefficient of the base frequency phase based on a ratio of the reference phase to the base frequency phase, and determine a weight coefficient of the second harmonic phase based on a ratio of the reference phase to the second harmonic phase; and weighted average the base frequency phase and the second harmonic phase based on the weight coefficient of the base frequency phase and the weight coefficient of the second harmonic phase, to obtain a real-time phase of the tissue.

According to a second aspect, an embodiment of the present application provides an electrosurgical system including: the electrosurgical generator according to the first aspect of the present application; and a cutter coupled to the electrosurgical generator, wherein the cutter includes two electrodes for clamping tissue; wherein energy is output to the tissue by the electrosurgical generator via the two electrodes of the cutter.

According to a third aspect, an embodiment of the present application provides a control method applied to the electrosurgical generator according to the first aspect of the present application or the electrosurgical system according to the second aspect of the present application. The method includes: executing a plurality of sub-processes sequentially after it is determined that the tissue has been effectively clamped by the two electrodes of the cutter; during each sub-process, determining at least one control parameter and at least one ending parameter of current sub-process based on at least one impedance parameter and at least one time parameter of the tissue; and outputting energy to the tissue based on the at least one control parameter of the current sub-process, and determining whether the current sub-process should be ended based on the at least one ending parameter of the current sub-process.

The technical solution provided by the embodiments of the present application can adaptively control a tissue closure process according to the type, size and state of the tissue. Among them, the tissue closure process consists of several repeated sub-processes; for each sub-process, a state of the tissue may be judge first, and a way of outputting energy to the tissue may be adjusted according to the judgment result. Therefore, for tissues that are easy to close or of smaller size, only fewer sub-processes are required to achieve closure, and the closure speed is fast; for tissues that are difficult to close or of larger size, multiple sub-processes will be used to ensure that the tissue is fully closed. Thereby, an energy output to the tissue may be dynamically and accurately controlled according to the closure state of the tissue, thereby improving a success rate of tissue closure and a bursting pressure of the tissue.

## BRIEF DESCRIPTION OF THE DRAWINGS

FIG. 1 is a schematic diagram of a structure of an electrosurgical system provided according to an embodiment of the present application;

FIG. 2 is a basic structural block diagram of an electrosurgical generator provided according to an embodiment of the present application;

FIG. 3 is a structural block diagram of an electrosurgical generator provided according to an embodiment of the present application;

FIG. 4 is a flow chart of a method for calculating tissue impedance and phase according to an embodiment of the present application;

FIG. 5 is a flow chart of a control method provided by an embodiment of the present application;

FIG. 6 is a flow chart of a tissue fusion stage according to an embodiment of the present application;

FIG. 7 is a power control graph during tissue closure process according to an embodiment of the present application;

FIG. 8 is a graph illustrating tissue impedance change during tissue closure process according to an embodiment of the present application.

## EMBODIMENTS

The technical solutions according to embodiments of the present application will be described clearly and completely as follows in combination with the drawings in the embodiments of the present application. Obviously, the described embodiments are only part of the embodiments of the present application, not all of the embodiments. Based on the embodiments in the present application, all other embodiments obtained by a person skilled in the art without creative work are within the scope of protection of this application.

The technical solution of the embodiment of the present application can be applied to endoscopic instruments, laparoscopic instruments or open instruments, to implement electrosurgical operations such as electrocutting, electrocoagulation, tissue closure, and blood vessel closure by applying energy to tissue parts. It is understandable that in instruments implemented by the technical solution of the embodiment of the present application, electrical connection and/or mechanical connection between the components may be different, and these differences are also within the protection scope of the embodiment of the present application.

An embodiment of the present application provides an electrosurgical system. The electrosurgical system may include an electrosurgical generator, and one or more surgical instruments that can be connected to the electrosurgical generator. The surgical instruments include, but are not limited to, bipolar electrosurgical forceps (also referred to as bipolar cutter), monopolar electrosurgical forceps (also referred to as monopolar cutter), monopolar active electrodes, return electrode, pedals, etc. The electrosurgical generator can apply energy based on a specific method, and supply the energy to a tissue site via one or more electrosurgical forceps connected thereto. In addition, the electrosurgical generator can also receive feedback signals from one or more electrosurgical forceps, and control the energy supplied by it according to the feedback signals.

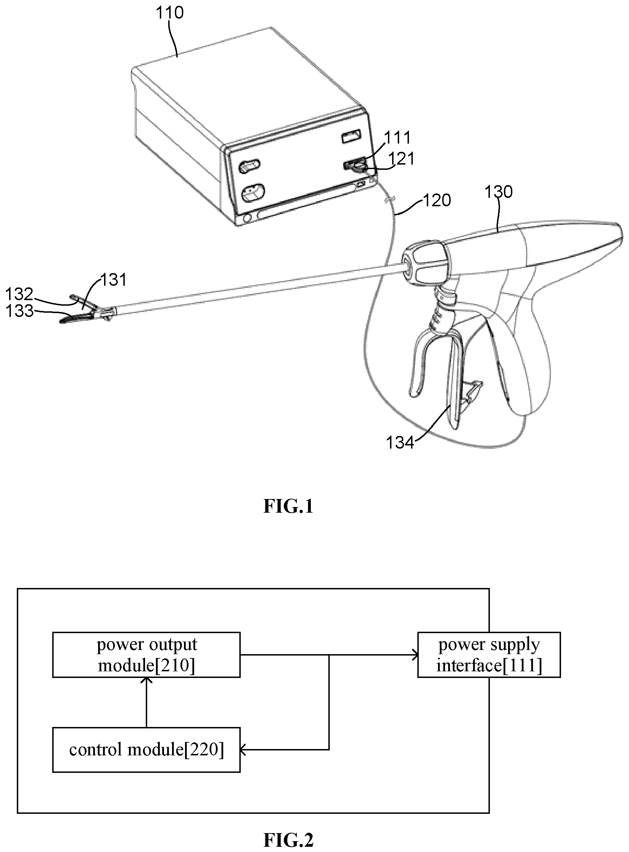

FIG. 1 is a schematic diagram of a structure of an electrosurgical system provided according to an embodiment of the present application. As shown in FIG. 1, the electrosurgical system includes: an electrosurgical generator 110, and an electrosurgical forceps 130 (e.g., a monopolar electrosurgical forceps or a bipolar electrosurgical forceps) connected to the electrosurgical generator 110 via a cable 120. The electrosurgical generator 110 is used to generate and control the energy output to the tissue site, and also includes one or more power supply interfaces 111. The electrosurgical forceps 130 include: a jaw 131, an electrode 132 and/or an electrode 133 provided at the jaw 131. Within the jaw 131, the electrode 132 and the electrode 133 can be connected to the cable 120 via a conductor (e.g., an internal wire, etc.). In addition, one end of the cable 120 further includes a power receiving terminal 121 matching with the power supply interface 111. When the power receiving terminal 121 is inserted into the power supply interface 111, the energy generated by the electrosurgical generator 110 can be output to the tissue site through the cable 120, the conductor inside the cutter, and the electrodes 132 and 133 of the jaws 131, thereby achieving effects such as electric cutting, electric coagulation, tissue closure, and blood vessel closure.

In one example, the electrosurgical system may specifically be a high-frequency electrosurgical cutter system. The electrosurgical generator 110 may be a high-frequency electrosurgical cutter generator (also referred to as a high-frequency electrosurgical cutter main unit) for outputting a high-frequency AC signal, and the electrosurgical forceps 130 may be a bipolar cutter comprising two electrodes. The jaws 131 of the bipolar cutter are designed to have a claw configuration with a clamping function, wherein the electrode 132 and the electrode 133 are oppositely arranged on the clamping surfaces of the claw configuration. When the user holds the handle 134 of the bipolar cutter and applies force, the jaws 131 of the bipolar cutter can effectively clamp the tissue and apply a relatively large pressure onto the tissue site. The high-frequency current generated by the high-frequency electrosurgical cutter generator can be output to the tissue clamped between the electrode 132 and the electrode 133 to close the tissue. In this way, the high-frequency current can be confined between the electrode 132 and the electrode 133, and will not flow through the surface of the human body, thereby ensuring a safer and more reliable operation. Tissue closure refers to a process of fusing the collagen elastin and matrix on both sides of the tissue via energy, so that they may form a fusion mass volume without obvious boundaries.

In one example, the high-frequency electrosurgical cutter system can work in high-frequency bipolar mode, which can be used to close arteries, veins and lymphatic vessels below 7 mm. Compared with an ordinary bipolar mode, the high-frequency bipolar mode outputs energy with high power and low voltage, with higher output current, and the energy may be reasonably output, thereby preventing tissue adhesion and carbonization; finally, with the high pressure exerted on the tissue by the claws of the cutter, it is possible to form a firm closure area.

FIG. 2 is a basic structural block diagram of an electrosurgical generator according to an embodiment of the present application.

As shown in FIG. 2, in one implementation, the electrosurgical generator may include a power output module 210 and a control module 220. The power output module 210 is connected to the power supply interface 111 of the electrosurgical generator, and is used to output energy to the cutter connected to the electrosurgical generator through the power supply interface 111. The control module 220 is used to sample the output signal of the power output module 210 to obtain a corresponding sampling signal. The sampling signal may include, for example, voltage and current output by the power output module 210. Then, the control module 220 may determine the real-time impedance of the tissue according to the sampling signals. Next, after it is determined that the tissue has been effectively clamped by the two electrodes of the cutter according to the real-time impedance of the tissue, the control module 220 may determine at least one control parameter based on a specific algorithm, wherein the control parameter includes but is not limited to one or more of the following parameters: output power, output voltage, output current, power curve, and stop impedance. Finally, the control module 220 may control the energy output to the tissue by the power output module 210 according to the above control parameters, thereby realizing an adaptive closed-loop control process for the entire electrosurgical system.

In an embodiment of the present application, the adaptive closed-loop control process for the electrosurgical system may be composed of several sub-processes that are repeatedly executed. Taking tissue closure as an example, each sub-process may include a state determination stage and a tissue fusion stage. In the state determination stage, the control module 220 may determine the closure state of the current tissue based on at least one impedance parameter and at least one time parameter of the tissue, based on some specific algorithms, and determine at least one control parameter and at least one ending parameter of the current sub-process. In the tissue fusion stage, the control module 220 may adjust the energy output by the power output module 210 to the tissue in real time based on control parameters of the current sub-process, and determine whether to end the current sub-process based on the at least one ending parameter of the current sub-process.

It is understandable that: the power output module 210 may include one or more power output circuits and one or more interface circuits, etc., in order to realize the function of outputting energy to the tissue; the control module 220 may include one or more output sampling circuits and one or more chips for executing algorithms and/or storing data, etc., in order to realize signal sampling, parameter calculation and control functions. It should be noted here that in different designs, the electrosurgical generator used to implement the various algorithms and functions involved in the embodiments of the present application may be designed to have different configurations; however, regardless of whether the configurations are the same or different, these designs all adopt the technical concepts of the embodiments of the present application, and therefore do not go beyond the protection scope of the embodiments of the present application.

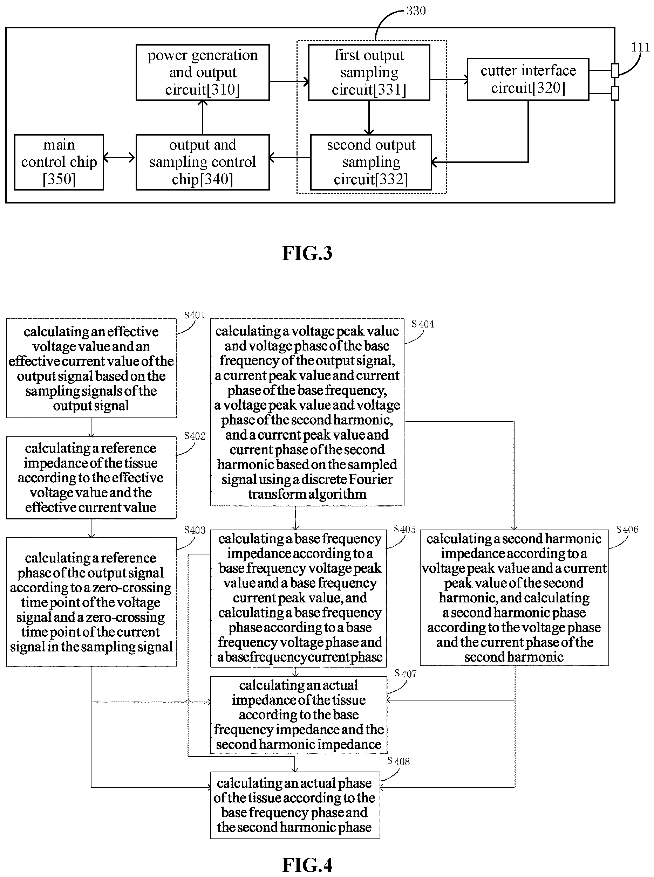

FIG. 3 is a structural block diagram of an electrosurgical generator according to an embodiment of the present application.

As shown in FIG. 3, in one implementation, the electrosurgical generator may be composed of a power generation and output circuit 310, a cutter interface circuit 320, a power supply interface 111, an output sampling module 330, an output and sampling control chip 340, and a main control chip 350. The power generation and output circuit 310 and the cutter interface circuit 320 may be used to carry out the function of the power output module 210 in FIG. 2, and the output sampling module 330, the output and sampling control chip 340, and the main control chip 350 may be used to carry out the function of the control module 220 in FIG. 2.

The power generation and output circuit 310 is connected to the power supply interface 111 of the electrosurgical generator through the cutter interface circuit 320, and is used to generate and output energy, such as high-frequency AC signals, under the control of the output and sampling control chip 340. When the two electrodes of the cutter clamp the tissue, the energy output by the power generation and output circuit 310 can be output to the tissue site through the cutter interface circuit 320, the power supply interface 111 and the cutter.

The output sampling module 330 may be provided at the output side of the power generation and output circuit 310, is composed of at least one output sampling circuit, and is used to sample the output signals of the power generation and output circuit 310, obtain corresponding sampling signals, and send the sampling signals to the output and sampling control chip 340. The sampling signals may include, for example, voltage and current output by the power generation and output circuit 310. FIG. 3 exemplarily shows two output sampling circuits, which are respectively referred to as a first output sampling circuit 331 and a second output sampling circuit 332 for easy distinction. Each output sampling circuit may include one or more sensors, including but not limited to: one or more voltage sensors, and/or one or more current sensors.

In one example, different output sampling circuits may include different types of sensors to obtain different types of sampling results. For example, the first output sampling circuit 331 may include a current sensor to perform current sampling on the output signal, and the second output sampling circuit 332 may include a voltage sensor to perform voltage sampling on the output signal.

It should be noted here that in different designs, the output sampling module 330 may be implemented in different ways, such as including more output sampling circuits or fewer output sampling circuits, including more types of sensors or including fewer types of sensors, and each output sampling circuit is configured to implement a sampling function different from the above examples. These designs do not exceed the protection scope of the embodiments of the present application.

The main control chip 350 is used to execute a software-controlled logic and algorithm of the electrosurgical system. For example, according to the real-time sampling signals of the output and sampling control chip 340, one or more parameters of the output signal are calculated, and the parameters may include, for example, impedance corresponding to the base frequency or second harmonic of the output signal, a phase difference between the voltage and the current, etc. Further, the main control chip 350 may generate at least one control parameter according to the algorithm based on one or more parameters of the output signal, wherein the control parameter includes but is not limited to one or more of the following parameters: output power, output voltage, output current, power curve, stop impedance, etc.

According to some embodiments, the main control chip 350 may include one or more processors/processing units. For example, the main control chip 350 may be: an ARM architecture processor ( Advanced RISC Machine, Advanced Reduced Instruction Set Computer), a digital signal processor (DSP), a field programmable gate array (FPGA), an application-specific integrated circuit (ASIC), a central processing unit (CPU), a graphics processing unit (GPU), a microcontroller unit (MCU ), etc. Among them, when the main control chip 350 is composed of multiple processors/processing units, different processing units/processing units may be independent devices, or they may be integrated into one chip, such as integrated into a system on a chip (SoC).

The output and sampling control chip 340 is used to send the sampling signals of the output sampling module 330 to the main control chip 350, and to perform real-time control on the power generation and output circuit 310, the first output sampling circuit 331, and the second output sampling circuit 332 according to the control parameters sent by the main control chip 350.

In one implementation, the output sampling circuit can sample the voltage and current of the output signal in real time. The output and sampling control chip 340 may configure a number of sampling points in each signal cycle of the output signal of each output sampling circuit.

In one example, when the electrosurgical system is implemented as a high-frequency electrosurgical cutter system, the frequency of the high-frequency signal outputted by the electrosurgical uncutter system may be between 450 kHz and 550 kHz. Accordingly, 64 sampling points or 128 sampling points may be included in each signal cycle for the output sampling circuit.

The embodiments of the present application do not specifically limit the number of sampling points of the output sampling circuit in each signal cycle. When implementing the present application, technicians can reasonably select the number of sampling points in each signal cycle according to the frequency of the output signal, the computing capability of the chip, the algorithm requirements, the requirements for the precision of a control on the output energy, etc., which do not exceed the protection scope of the embodiments of the present application.

According to some embodiments, the output and sampling control chip 340 may include one or more processors/processing units. For example, the output and sampling control chip 340 may be: an ARM architecture processor, DSP, FPGA, ASIC, CPU, GPU, MCU, etc. When the output and sampling control chip 340 is composed of multiple processors/processing units, different processing units/processing units may be independent devices or integrated into one chip, such as integrated into an SoC.

According to some implementations, the functions of the main control chip 350 and the output and sampling control chip 340 may also be implemented by the same chip. For example, the electrosurgical generator may only include the main control chip 350, and the algorithm and signal transmission function of the sampling control chip may be implemented by the main control chip 350 at the same time.

According to some embodiments, the electrosurgical generator may further include one or more memories. The memory may be used to store the algorithm program executed by the main control chip 350 and/or the output and sampling control chip 340, as well as to store data, cache, etc. generated during the execution of the algorithm program, and may also store the sampling results of the output sampling module 330, etc. The memories may be independent devices, or may be integrated with the processor or packaged in the same chip to become a part of the main control chip 350 and/or the output and sampling control chip 340. The memory may include, for example, volatile memory, such as dynamic random access memory (DRAM), static random access memory (SRAM), etc.; the memory may also include non-volatile memory (NVM), such as electrically erasable programmable read-only memory (EEPROM), read-only memory (ROM), flash memory, etc.

According to some embodiments, the electrosurgical generator may further include one or more switches, one or more buttons, etc. For example, a power switch, a signal output switch, a working mode button, etc., may be used, which are not limited in the embodiments of the present application.

According to some embodiments, the electrosurgical generator further includes one or more external pedals. When the user steps on the pedals, the electrosurgical generator can perform corresponding functions, such as starting a tissue closure process, outputting an initial signal, and the like.

According to some embodiments, the electrosurgical generator may further include one or more displays, which may be used to display information of the electrosurgical generator output signal, such as: the power, frequency, tissue impedance, tissue phase, notification information of successful or failed tissue closure, notification information of the cutter effectively clamping the tissue or not effectively clamping the tissue, etc. The embodiments of the present application are not limited to this.

According to some embodiments, the electrosurgical generator may further include one or more playback devices, such as a speaker, a buzzer, etc., which are used to play sound signals to the user to convey corresponding information, such as: notification information of successful tissue closure or failed tissue closure, notification information of the cutter effectively clamping the tissue or the cutter not effectively clamping the tissue, etc. The embodiments of the present application are not limited to this.

During electrosurgery, the actual surgical environment is very complex, and the circuit and tissue parts will have varying parasitic inductance and capacitance due to the influence of temperature, blood, body fluid penetration, surrounding tissue, cutter status, etc. The presence of parasitic inductance and capacitance will increase the difficulty in calculating tissue impedance and phase, resulting in an inaccuracy of the tissue impedance and phase calculated with traditional single algorithms.

To this end, the present application provides a method for more accurately calculating tissue impedance and phase. The method can be applied to the electrosurgical generator or electrosurgical system provided in the present application, or to other devices or systems, which is not limited in the present application.

In one implementation, the method for calculating tissue impedance and phase can be implemented by a control module (e.g., a main control chip and/or an output and sampling control chip) in an electrosurgical generator executing a corresponding algorithm; in the other words, the control module (e.g., a main control chip and/or an output and sampling control chip) may be configured to execute the various steps of the method.

FIG. 4 is a flow chart of a method for calculating tissue impedance and phase according to an embodiment of the present application. As shown in FIG. 4, in one example, the method may include the following steps S401 -S408:
Step S401 : calculating an effective voltage value and an effective current value of the output signal based on the sampling signals of the output signal.

In one implementation, the main control chip can calculate the effective voltage value and effective current value of the output signal using an effective value algorithm based on the voltage values and current values of multiple sampling points collected by the output sampling circuit in one or more cycles.

For example, the effective value of voltage can be calculated using the following formula:

$$U_{rms} = \sqrt{\frac{\sum_1^N U_t^2}{N}}$$

Among them, $U_{rms}$ represents the effective value of voltage, N represents the number of sampling points, Ut represents the voltage value of the t-th sampling point, and $t \in [1, N]$.

For example, the effective value of the current can be calculated using the following formula:

$$I_{rms} = \sqrt{\frac{\sum_1^N I_t^2}{N}}$$

Among them, $I_{rms}$ represents the effective value of current, N represents the number of sampling points, It represents the current value of the t-th sampling point, and $t \in [1, N]$.

Step S402, calculating a reference impedance of the tissue according to the effective voltage value and the effective current value.

Exemplarily, the reference impedance of the tissue can be calculated using the following formula:

$$Z_{ref} = \frac{U_{rms}}{I_{rms}}$$

Among them, $Z_{ref}$ represents the reference impedance, $U_{rms}$ represents the effective value of voltage, and $I_{rms}$ represents the effective value of current.

Step S403, calculating a reference phase of the output signal with a zero-crossing comparison method according to a zero-crossing time point of the voltage signal and a zero-crossing time point of the current signal in the sampling signal.

Exemplarily, the reference phase of the output signal can be calculated using the following formula:

$$\vartheta_{ref} = 2\pi * \frac{t_u - t_I}{T}$$

Among them, $\vartheta_{ref}$ represents the reference phase, $t_u$ represents the zero-crossing time point of the voltage signal, $t_I$ represents the zero-crossing time point of the current signal, and T represents the period of the output signal.

Step S404, calculating a voltage peak value and voltage phase of the base frequency of the output signal, a current peak value and current phase of the base frequency, a voltage peak value and voltage phase of the second harmonic, and a current peak value and current phase of the second harmonic based on the sampled signal using a discrete Fourier transform algorithm.

It should be noted that for an electrosurgical system, the tissue clamped by the cutter forms a nonlinear load. Therefore, the voltage signal or current signal output to the tissue contains not only waveform of the base frequency, but also waveforms of harmonics (whose frequencies are integral multiples of the base frequency), such as a second harmonic, a third harmonic, etc. Therefore, in order to more accurately calculate the real-time impedance and real-time phase of the tissue, the influence of harmonics needs to be considered.

In step S404, the discrete Fourier transform algorithm that can be used includes but is not limited to a fast Fourier transform ( FFT ) algorithm or a Goertzel algorithm, etc., which is not limited in the embodiment of the present application.

Step S405, calculating a base frequency impedance according to a base frequency voltage peak value and a base frequency current peak value, and calculating a base frequency phase according to a base frequency voltage phase and a base frequency current phase.

The base frequency impedance is the ratio of the voltage peak value to the current peak value of the base frequency of the output signal, which is calculated using the following formula:

$$Z_1 = U_1 / I_1$$

Among them, $Z_1$ represents the base frequency impedance, $U_1$ represents the voltage peak value of the base frequency, and $I_1$ represents the current peak value of the base frequency.

The base frequency phase is the phase difference between the voltage phase of the base frequency and the current phase of the base frequency of the output signal, which is calculated using the following formula:

$$\vartheta_1 = \vartheta_{v1} - \vartheta_{I1}$$

Among them, $\vartheta_1$ represents the base frequency phase, $\vartheta_{v1}$ represents the voltage phase of the base frequency, and $\vartheta_{I1}$ represents the current phase of the base frequency.

Step S406, calculating a second harmonic impedance according to a voltage peak value of the second harmonic and a current peak value of the second harmonic, and calculating a second harmonic phase according to the voltage phase and the current phase of the second harmonic.

The second harmonic impedance is the ratio of the voltage peak value to the current peak value of the second harmonic of the output signal, which is calculated using the following formula:

$$Z_2 = U_2 / I_2$$

Among them, $Z_2$ represents the second harmonic impedance, $U_2$ represents the voltage peak value of the second harmonic, and $I_2$ represents the current peak value of the second harmonic.

The second harmonic phase is a phase difference between the voltage phase of the second harmonic and the current phase of the second harmonic of the output signal, which is calculated using the following formula:

$$\vartheta_2 = \vartheta_{v2} - \vartheta_{I2}$$

Among them, $\vartheta_2$ represents the second harmonic phase, $\vartheta_{v2}$ represents the voltage phase of the second harmonic, and $\vartheta_{I2}$ represents the current phase of the second harmonic.

Step S407, calculating an actual impedance of the tissue according to the base frequency impedance and the second harmonic impedance.

In one implementation, the actual impedance of the tissue can be obtained by taking a weighted average of the base frequency impedance and the second harmonic impedance, which can be specifically calculated using the following formula:

$$Z = w_{Z1}Z_1 + w_{Z2}Z_2$$

Among them, Z represents the actual impedance of the tissue, $Z_1$ represents the base frequency impedance, $w_{Z1}$ represents the weight coefficient of the base frequency impedance, $Z_2$ represents the second harmonic impedance, and $w_{Z2}$ represents the weight coefficient of the second harmonic impedance.

In one implementation, the weight coefficient of the base frequency impedance may be determined according to the ratio of the base frequency impedance to the reference impedance.

Exemplarily, based on a ratio of the base frequency impedance to the reference impedance, a mapping $f_{Z1}$ from the ratio of the base frequency impedance to the reference impedance to the weight coefficient of the base frequency impedance may be constructed via an algorithm, and ratios of the base frequency impedance to the reference impedance are mapped into an interval of the values of the weight coefficient by the mapping function $f_{Z1}$ example, thereby obtaining the weight coefficient of the base frequency impedance. The mapping function $f_{Z1}$ can be expressed as:

$$w_{Z1} = f_{Z1}\left(\frac{Z_1}{Z_{ref}}\right)$$

Among them, $w_{Z1}$ represents the weight coefficient of the base frequency impedance, $Z_1$ represents the base frequency impedance, and $Z_{ref}$ represents the reference impedance.

In one implementation, the weight coefficient of the second harmonic impedance may be determined according to the ratio of the second harmonic impedance to the reference impedance.

Exemplarily, based on a ratio of the second harmonic impedance to the reference impedance, a mapping $f_{Z2}$ from the ratio of the second harmonic impedance to the reference impedance to the weight coefficient of the second harmonic impedance can be constructed by an algorithm, and ratios of the second harmonic impedance to the reference impedance are mapped into an interval of the values of the weight coefficient by the mapping function $f_{Z2}$ example, thereby obtaining the weight coefficient of the second harmonic impedance. The mapping function $f_{Z2}$ can be expressed as:

$$w_{Z2} = f_{Z2}\left(\frac{Z_2}{Z_{ref}}\right)$$

Among them, $w_{Z2}$ represents the weight coefficient of the second harmonic impedance, $Z_2$ represents the second harmonic impedance, and $Z_{ref}$ represents the reference impedance.

Step S408, calculating an actual phase of the tissue according to the base frequency phase and the second harmonic phase.

In one implementation, the actual phase of the tissue can be obtained by taking a weighted average of the base frequency phase and the second harmonic phase, which can be specifically calculated using the following formula:

$$\vartheta = w_{\vartheta 1}\vartheta_1 + w_{\vartheta 2}\vartheta_2$$

Among them, $\vartheta$ represents the actual phase of the tissue, $\vartheta_1$ represents the base frequency phase, $w_{\vartheta 1}$ represents the weight coefficient of the base frequency phase, $\vartheta_2$ represents the second harmonic phase, and $w_{\vartheta 2}$ represents the weight coefficient of the second harmonic phase.

In one implementation, the weight coefficient of the base frequency phase may be determined according to the ratio of the base frequency phase to the reference phase.

Exemplarily, based on a ratio of the base frequency phase to the reference phase, a mapping from the ratio of the base frequency phase to the reference phase to the weight coefficient of the base frequency phase can be constructed by an algorithm, and the ratios of the base frequency phase to the reference phase are mapped into an interval of the values of the weight coefficient by the mapping function example, thereby obtaining the weight coefficient of the base frequency phase. The mapping function $f_{\vartheta 1}$ can be expressed as:

$$w_{\vartheta 1} = f_{\vartheta 1}\left(\frac{\vartheta_1}{\vartheta_{ref}}\right)$$

Among them, $w_{\vartheta 1}$ represents the weight coefficient of the base frequency phase, $\vartheta_1$ represents the base frequency phase, and $\vartheta_{ref}$ represents the reference phase.

In one implementation, the weight coefficient of the second harmonic phase may be determined according to a ratio of the second harmonic phase to a reference phase.

Exemplarily, based on a ratio of the second harmonic phase to the reference phase, a mapping from the ratio of the second harmonic phase to the reference phase to the weight coefficient of the second harmonic phase can be constructed by an algorithm, and the ratios of the second harmonic phase to the reference phase are mapped into an interval of the values of the weight coefficient by the mapping function example, thereby obtaining the weight coefficient of the second harmonic phase. The mapping function $f_{\vartheta 2}$ can be expressed as:

$$w_{\vartheta 2} = f_{\vartheta 2}\left(\frac{\vartheta_2}{\vartheta_{ref}}\right)$$

Among them, $w_{\vartheta 2}$ represents the weight coefficient of the second harmonic phase, $\vartheta_2$ represents the second harmonic phase, and $Z_{ref}$ represents the reference phase.

It can be understood that the main control chip can calculate the actual impedance and actual phase of the tissue in real time based on the voltage values and current values of multiple sampling points collected by the output sampling circuit in one or more cycles. In this way, the actual impedance and phase calculated by the main control chip are the real-time impedance and real-time phase of the tissue.

According to the above method, the electrosurgical generator calculates the reference impedance and reference phase of the tissue based on an effective value algorithm and a zero-crossing comparison algorithm, and calculates the base frequency impedance, base frequency phase, second harmonic impedance, and second harmonic phase of the tissue via a discrete Fourier transform algorithm, and finally calculate the real-time impedance and real-time phase of the tissue via a weighted average method. Therefore, compared with a traditional method using a single algorithm in calculation, the method for calculating tissue impedance and phase according to the embodiment of the present application can calculate the real-time impedance and real-time phase of the tissue in a more accurate way.

According to some embodiments, the electrosurgical system may also take into account the third harmonic, or even higher harmonics, when calculating the actual impedance and actual phase of the tissue.

Then, the actual impedance of the tissue can be calculated by the following formula:

$$Z = \sum_{i=1}^{N} w_{zi}\, Z_i$$

Where Z represents the actual impedance of the tissue, N represents the highest order of harmonics considered in the calculation, $w_{zi}$ represents the weight coefficient of the i-th harmonic impedance, and $Z_i$ represents the i-th harmonic impedance (when i = 1, it represents the base frequency impedance).

The weight coefficient of the i-th harmonic impedance can be obtained by the following mapping function $f_{Zi}$:

$$w_{Zi} = f_{Zi}\left(\frac{Z_i}{Z_{ref}}\right)$$

Among them, $w_{Zi}$ represents the weight coefficient of the i-th harmonic impedance, $Z_i$ represents the i-th harmonic impedance, and $Z_{ref}$ represents the reference impedance.

In addition, the actual phase of the tissue can be calculated using the following formula:

$$\vartheta = \sum_{i=1}^{N} w_{\vartheta i}\, \vartheta_i$$

Among them, $\vartheta$ represents the actual phase of the tissue, N represents the highest order of harmonics considered in the calculation, $w_{\vartheta i}$ represents the weight coefficient of the i-th harmonic phase, and $\vartheta_i$ represents the i-th harmonic phase (when i = 1, it represents the base frequency phase).

The weight coefficient of the i-th harmonic phase can be obtained by the following mapping function $f_{\vartheta i}$:

$$w_{\vartheta i} = f_{\vartheta i}\left(\frac{\vartheta_i}{\vartheta_{ref}}\right)$$

Among them, $w_{\vartheta i}$ represents the weight coefficient of the i-th harmonic phase, $\vartheta_i$ represents the i-th harmonic phase, and $\vartheta_{ref}$ represents the reference phase.

The present application also provides a control method, which can be applied to the electrosurgical generator or electrosurgical system provided in the present application, or to other devices or systems, which is not limited by the present application.

In one implementation, the control method can be implemented by a control module (e.g., the main control chip and/or the output and sampling control chip) in an electrosurgical generator executing a corresponding algorithm; in other words, the control module (e.g., a main control chip and/or an output and sampling control chip) is configured to execute respective step of the method.

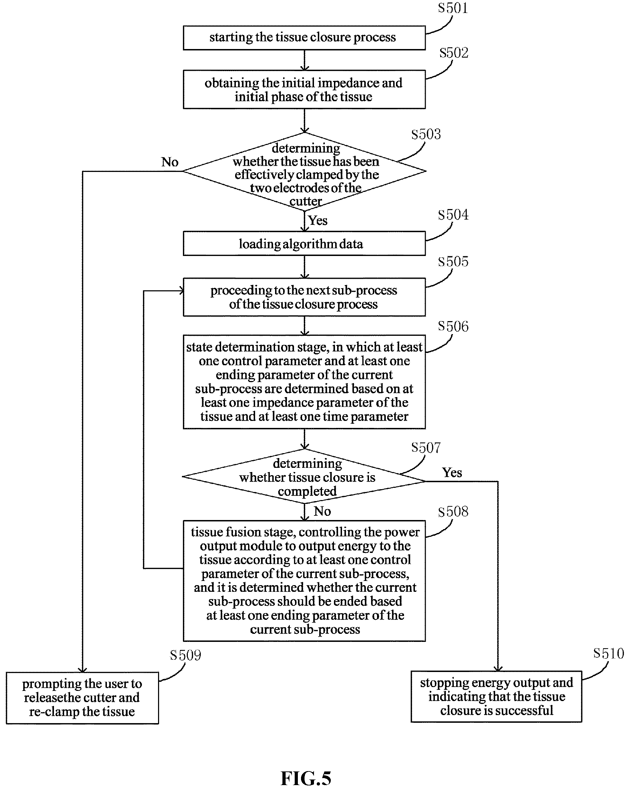

Fig. 5 is a flow chart of a control method according to an embodiment of the present application. In conjunction with Fig. 5, the steps of the control method will be exemplarily described below by taking an electrosurgical system applying the control method to implement tissue closure as an example.

As shown in FIG. 5, in one example, the method may include the following steps S501 - S510:

Step S501, starting the tissue closure process.

In a specific implementation, a user (e.g., a physician performing a tissue closure operation) can start the tissue closure process by pressing a manual switch on the cutter or stepping on a corresponding foot pedal.

After the tissue closure process begins, the electrosurgical generator starts to output an initial signal, which can also be called a small signal. The initial signal can be a low-power high-frequency AC signal, whose power range can be, for example, between 1 W and 10 W, and whose duration can be between 10 ms and 30 ms.

In one implementation, the initial signal may have a power of 8 W, a duration of 100 ms, and a frequency of 450 kHz.

In the embodiment of the present application, the initial signal can be used in the electrosurgical generator to perform signal sampling and calculate the initial impedance and initial phase of the tissue. Then, when implementing this method, the technician can determine the power, duration, frequency and other parameters of the initial signal according to a sampling capability of the output sampling circuit, data requirements of the main control chip for calculating the initial impedance and initial phase, etc., to ensure that the power and frequency of the initial signal are within the sampling range of the output sampling circuit, and the data collected by the output sampling circuit within the duration of the initial signal can meet the data requirements for calculating the initial impedance and initial phase.

Step S502, obtaining the initial impedance and initial phase of the tissue.

In a specific implementation, the electrosurgical system may calculate the initial impedance and initial phase of the tissue based on the sampling results on the initial signal according to the method provided in the above steps S401 to S408, which will not be described in detail here.

Step S503, determining whether the tissue has been effectively clamped by the two electrodes of the cutter.

If yes, execute step S504; and if no, execute step S509. In step S509, the electrosurgical system may prompt the user to release the cutter and re-clamp the tissue via voice message, information displayed on a display screen, or indicator lights.

Generally speaking, in actual surgery, different tissue impedances and phases will occur when the cutter clamps tissues of different types or sizes, and there will be different correlations between the tissue impedance and tissue phase. In addition, a magnitude of the tissue impedance is also related to factors such as a clamping-state of the cutter on the tissue and the surrounding environment of the tissue. For example: when inadequate tissue is clamped between the two electrodes of the cutter, or when liquid exists around the tissue, the tissue impedance will be small; when excessive tissue is clamped between the two electrodes of the cutter, or when the circuit is open, the tissue impedance will be large. Therefore, the electrosurgical system can determine whether the cutter has effectively clamped on the tissue based on the initial impedance and initial phase of the tissue.

In one implementation, a technician can pre-establish a query table based on the correspondence between impedance and phase. The query table can be pre-stored in the memory of the electrosurgical generator. The query table can specifically record the correspondence between different tissue phases and the upper impedance threshold and the lower impedance threshold. In this way, the electrosurgical system can obtain an upper impedance threshold $Z_{upper}$ and a lower impedance threshold $Z_{lower}$ corresponding to the initial impedance $Z_0$ by looking up the table, then determine whether the initial impedance $Z_0$ falls between the upper impedance threshold $Z_{upper}$ and the lower impedance threshold $Z_{lower}$; if the initial impedance $Z_0$ is between the upper impedance threshold $Z_{upper}$ and the lower impedance threshold $Z_{lower}$, it means that the tissue is effectively clamped by the two electrodes of the cutter; if the initial impedance $Z_0$ is higher than the upper impedance threshold $Z_{upper}$, it means that there is too much tissue clamped between the two electrodes of the cutter or there is an open circuit; if the initial impedance $Z_0$ is lower than the lower impedance threshold $Z_{lower}$, it means that there is too little tissue clamped between the two electrodes of the cutter, or there is liquid around the tissue.

In this way, the electrosurgical system can determine an impedance range by looking up a table based on the different correlations between tissue impedance and phase, and then determine whether the tissue has been effectively clamped by the cutter in a more accurate way by judging whether the initial impedance falls within the impedance range.

Step S504, loading algorithm data.

In a specific implementation, after confirming that the tissue has been effectively clamped by the two electrodes of the cutter, the electrosurgical system can start loading algorithm data of the tissue closure process. The algorithm data generally includes a program code and corresponding control parameters. The algorithm data can be stored in a memory or in the cloud. Then, the method proceeds to the adaptive tissue closure process.

Step S505, proceeding to the next sub-process of the tissue closure process.

Unlike a traditional tissue closure process, the tissue closure process of the embodiment of the present application consists of multiple sub-processes. In each sub-process, the electrosurgical system can determine whether the tissue closure is completed based on the measured tissue impedance, and determine the parameters for outputting energy to the tissue during the current sub-process The reason why the embodiment of the present application adopts such a design is that: the types of tissue and environment are relatively complex. For example, there may be too much liquid around the jaws 131 at the beginning, which will affect the impedance value measured; therefore, the parameters determined only based on the state of the tissue at a certain moment or during a certain stage may have relatively large randomness and errors, increasing the possibility of tissue closure failure. By dividing the tissue closure process into multiple sub-processes, the embodiment of the present application can achieve a more accurate judgment of the tissue state and a more accurate control on the energy output.

It should be noted here that, at the beginning of the tissue closure process, the "next sub-process" refers to the first sub-process of the tissue closure process.

Each sub-process may have a predetermined maximum duration, which may be between 100 ms and 3000 ms, and is not specifically limited here.

In a feasible implementation, the maximum duration of each sub-process is preferably 2000 ms.

In one implementation, each sub-process includes a state determination stage and a tissue fusion stage, wherein the state determination stage corresponds to the following steps S506 and S507, and the tissue fusion stage corresponds to the following step S508.

Step S506, state determination stage, in which at least one control parameter and at least one ending parameter of the current sub-process are determined based on at least one impedance parameter of the tissue and at least one time parameter.

In each sub-process, the duration of the state determination phase may be between 10 ms and 300 ms. The electrosurgical system may output energy in a constant manner during the state determination phase, and the power output may be between 1 W and 10 W.

In one implementation, the electrosurgical system can determine the control parameters of the current sub-process based on the minimum impedance of the tissue in the previous sub-process, the duration of the previous sub-process, and other parameters, so as to achieve a real-time control and a reasonable energy output in each sub-process. If it is the first sub-process, the control parameters of the current sub-process can be determined based on the initial impedance and the maximum duration of the sub-process.

The control parameter of the current sub-process may be at least one power control curve parameter of the current sub-process in the tissue fusion stage, such as a rising slope of the power control curve, etc. The power control curve determined according to the parameter includes but is not limited to a linear curve, a polynomial curve, an exponential curve, etc., which is not limited in the embodiment of the present application.

In a preferred implementation, the power control curve P(t) may be a curve of second order based on time t-power P. For example:

$$\mathrm{P}(t) = a * t^2 + b * t + c$$

Among them, a, b, c are the coefficients of the second order curve. The coefficients can be determined by parameters such as the minimum impedance of the tissue in the previous sub-process and the duration of the previous sub-process. For example:

$$\begin{cases} a = f_a(Z_{lastmin}, T_{last}) \\ b = f_b(Z_{lastmin}, T_{last}) \\ c = f_c(Z_{lastmin}, T_{last}) \end{cases}$$

Wherein: $Z_{lastmin}$ represents the minimum impedance of the tissue in the previous sub-process; $T_{last}$ represents the duration of the previous sub-process; $f_a, f_b, f_c$ are respective mapping functions used for obtaining each coefficient a, b, c according to $Z_{lastmin}$ and $T_{last}$, and are not specifically limited in the embodiment of the present application.

Furthermore, in each sub-process, the electrosurgical system can also determine at least one end judgment parameter of the current sub-process according to at least one impedance parameter. The at least one impedance parameter includes but is not limited to the minimum impedance of the tissue in the previous sub-process, the minimum impedance of the tissue in all sub-processes before the current sub-process, the initial impedance of the tissue, and other parameters; the at least one end judgment parameter includes but is not limited to the ending impedance of the current sub-process and the ending impedance at tissue closure.

In a specific implementation, the electrosurgical system may determine the ending impedance of the current sub-process according to the minimum impedance of the tissue in the previous sub-process.

In a feasible implementation, the ending impedance of the current sub-process can be calculated by the following function:

$$Z_{localend} = \alpha * Z_{lastmin} + Z_{adapt}$$

The function is a linear function, where: $Z_{localend}$ represents the ending impedance of the current sub-process, $Z_{lastmin}$ represents the minimum impedance of the tissue in the previous sub-process, $Z_{adapt}$ is a bias impedance; wherein the bias impedance is a fixed value, or a value that changes with the sub-process, and $\alpha$ is a fixed coefficient.

In a specific implementation, the electrosurgical system can determine the ending impedance at tissue closure according to the minimum impedance of the tissue in the previous sub-process, the minimum impedance of the tissue in all sub-processes before the current sub-process, and the initial impedance of the tissue.

In a feasible implementation, the ending impedance at tissue closure is a weighted average of the minimum impedance of the tissue in the previous sub-process, the minimum impedance of the tissue in all sub-processes before the current sub-process, and the initial impedance of the tissue. Specifically, it can be calculated by the following function:

$$Z_{end} = \beta_1 * Z_{lastmin} + \beta_2 * Z_{min} + \beta_3 * Z_0$$

Wherein: $Z_{end}$ represents the ending impedance at tissue closure, $Z_{lastmin}$ represents the minimum impedance of the tissue in the previous sub-process, $Z_{min}$ represents the minimum impedance of the tissue in all sub-processes before the current sub-process, $Z_0$ represents the initial impedance of the tissue, $\beta_1, \beta_2, \beta_3$ are weighting coefficients, and the embodiment of the present application does not specifically limit the values of the weighting coefficients.

In this way, during the state determination stage of each sub-process, the electrosurgical system can dynamically update the ending impedance of the current sub-process and the ending impedance at tissue closure based on the minimum impedance of the tissue in the previous sub-process, the minimum impedance of the tissue in all sub-processes before the current sub-process, the initial impedance of the tissue, etc., thereby achieving accurate judgment on whether each sub-process is completed and whether the tissue closure process is completed.

Step S507, determining whether tissue closure is completed.

If not, execute step S508. If yes, execute step S510. In step S510, the electrosurgical system may stop outputting energy and indicate that the tissue closure is successful by means of voice messages, information displayed on a display screen, or indicator light information, etc.

In step S507, the electrosurgical system can determine whether tissue closure is completed based on the numerical relationship between the ending impedance at tissue closure and the real-time impedance of the tissue. If the real-time impedance of the tissue is greater than the ending impedance at tissue closure, it is determined that the tissue closure is successful/complete; if the real-time impedance of the tissue is less than or equal to the ending impedance at tissue closure, it is determined that the tissue closure is not successful/complete.

In Step S508, also referred to as tissue fusion stage, the power output module is controlled to output energy to the tissue according to at least one control parameter of the current sub-process, and it is determined whether the current sub-process should be ended based at least one ending parameter of the current sub-process. When it is determined that the current sub-process should be ended, the process proceeds to step S505 in case that the tissue closure process has not timed out.

In a specific implementation, the electrosurgical system can output energy to the tissue according to the power control curve determined in the state determination stage of the current sub-process, and determine whether the ending condition of the current sub-process (which can also be described as the ending condition of the tissue fusion stage) is met. If the ending condition of the current sub-process is met, the system stops outputting energy to the tissue according to the power control curve and ends the current sub-process. The ending condition of the current sub-process includes but is not limited to: the real-time impedance of the tissue is greater than the ending impedance of the current sub-process, or the duration of outputting energy to the tissue according to the control parameters of the current sub-process is greater than a preset timeout period (i.e., the tissue fusion stage times out), or the duration of the current sub-process is greater than the preset maximum duration of the sub-process (i.e., the sub-process times out).

Among them, the timeout period of the tissue fusion stage can be between 500 ms and 3000 ms, which is not limited in the embodiment of the present application.

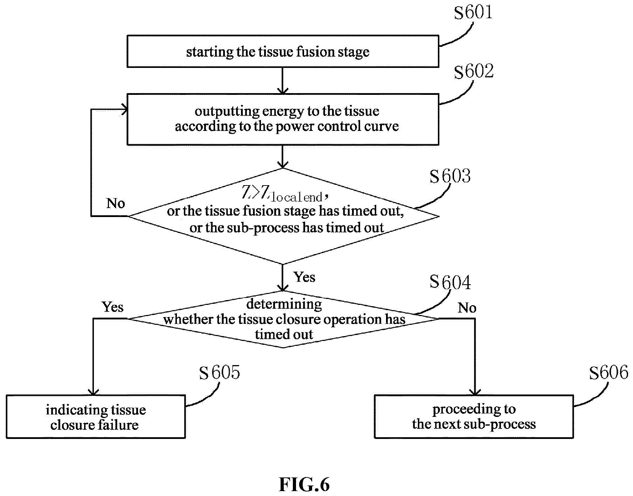

FIG. 6 is a flow chart of the tissue fusion stage according to an embodiment of the present application.

As shown in FIG. 6, in one implementation, the tissue fusion stage may include the following steps S601 - S606.

Step S601: start the tissue fusion stage.

Step S602: output energy to the tissue according to the power control curve.

In a specific implementation, the electrosurgical generator can adjust the power of the energy output to the tissue in real time according to the power control curve to close the tissue.

Step S603: determine whether the real-time impedance of the tissue is greater than the ending impedance of the current sub-process, or whether the duration of the tissue fusion stage is greater than the pre-set timeout period, or whether the duration of the previous sub-process is greater than the pre-set sub-process maximum duration. If yes, the process proceeds to step S604; if not, the process continues to output energy to the tissue according to the power control curve.

Step S604: determine whether the tissue closure operation has timed out.

If yes, the process proceeds to step S605. If no, the process proceeds to step S606.

Step S605: indicate tissue closure failure.

In a specific implementation, the electrosurgical system may indicate tissue closure failure via voice messages, information displayed on a display screen, or indicator light information, etc., which is not limited in this embodiment of the present application.

Step S606: proceed to the next sub-process.

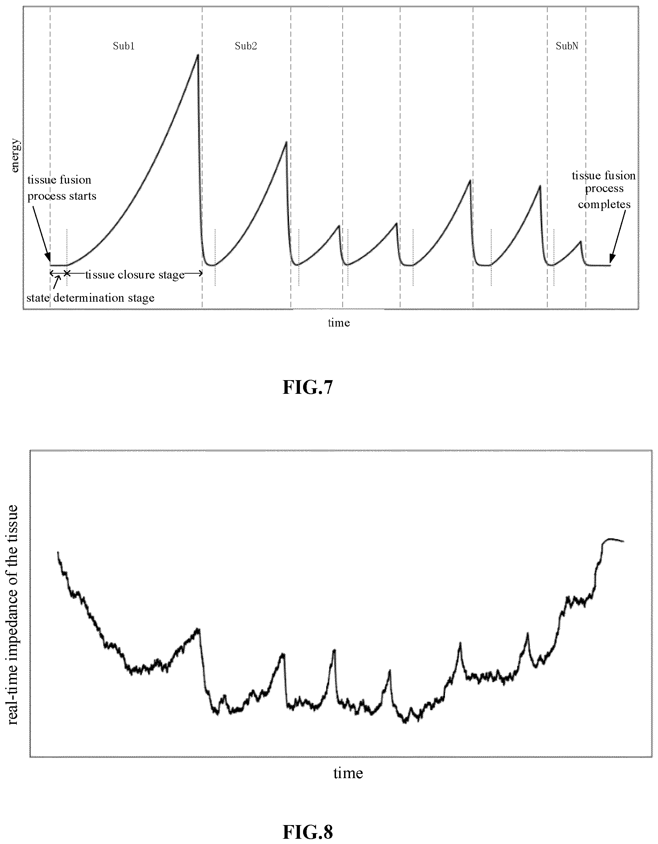

FIG. 7 is a power control graph during the tissue closure process provided by an embodiment of the present application.

Referring to FIG. 7, the power control graph illustrates how an output energy of the electrosurgical system changes over time during multiple sub-processes (e.g., Sub 1, Sub 2 ~ Sub N ). As can be seen from FIG. 7, in each sub-process, the power control curve includes two states: a first state where the power output is low and stable, corresponding to the state determination stage of the sub-process; a second state where the power output increases rapidly, corresponding to the tissue fusion stage of the sub-process. In addition, since the power control curve, ending impedance, etc. of each sub-process are determined based on parameters such as the minimum impedance of the tissue in the previous sub-process and the duration of the previous sub-process, therefore, in different sub-processes, the rising slope and peak value of the power control curve, as well as the duration of the sub-process are also continuously and dynamically adjusted based on these parameters. In this way, the electrosurgical system can dynamically and accurately control the energy output to the tissue in each sub-process, thereby improving a success rate of tissue closure and increasing a bursting pressure of the tissue.

FIG. 8 is a graph illustrating a tissue impedance change during tissue closure according to an embodiment of the present application.

According to current research, tissue impedance is generally at its lowest value during the tissue fusion stage. As can be seen from FIG. 8, via a series of sub-processes, tissue impedance can be maintained at a relatively low value for a relatively long time period; by this, a more complete tissue fusion can be achieved, and the fused tissue has a higher bursting pressure.

It can be seen from the above technical solutions that the embodiments of the present application provide an electrosurgical generator, an electrosurgical system and a control method thereof, which can adaptively control the tissue closure process according to the type, size and state of the tissue. The tissue closure process consists of a number of repeated sub-processes; for each sub-process, a state of the tissue may be judge first, and a way of outputting energy to the tissue may be adjusted according to the judgment result. Therefore, for tissues that are easy to close or of smaller size, only fewer sub-processes are required to achieve closure, and the closure speed is fast; for tissues that are difficult to close or of larger size, multiple sub-processes will be used to ensure that the tissue is fully closed to achieve a sufficiently high closure bursting pressure.

The technical solution provided according to the embodiments of the present application can be extended to other energy-based instruments or methods, including but not limited to ultrasound, laser, microwave and tissue cryopreservation. It is understandable that when the type of energy supply is different, the tissue parameters used to determine the energy output mode will also change accordingly. For example, when electrosurgical energy is employed to close the tissue, the tissue parameter can be an impedance parameter. When thermal energy, ultrasound, light waves, microwaves, etc. are used to treat tissue, the tissue parameter can be temperature, density, opacity, etc., which is not limited in the embodiment of the present application

The above specific implementation methods further illustrate the purpose, technical solutions and beneficial effects of the present invention in detail. It should be understood that the above are only specific implementation methods of the present invention and are not used to limit the scope of protection of the present invention. Any modifications, equivalent substitutions, improvements, etc. made on the basis of the technical solutions of the present invention should be included in the scope of protection of the present invention.

**Claims**

1.  An electrosurgical generator, **characterized in that** it comprises:

    a power output module configured to output energy to a tissue via two electrodes of a cutter; and
    a control module that is configured to:

    determine at least one impedance parameter of the tissue based on sampling signals of the output energy; and
    execute a plurality of sub-processes sequentially after it is determined that the tissue is effectively clamped between the two electrodes of the cutter;
    wherein during each sub-process,

    at least one control parameter and at least one ending parameter of the current sub-process is determined based on the at least one impedance parameter of the tissue and at least one time parameter; and
    the power output module is controlled to output energy to the tissue according to the at least one control parameter of the current sub-process, and it is determined whether the current sub-process should be ended according to the at least one ending parameter of the current sub-process.

2.  The electrosurgical generator according to claim 1, **characterized in that**
    the control module is configured to: in each sub-process, determine at least one control parameter of current sub-process according to a minimum impedance of the tissue in a previous sub-process and a duration of the previous sub-process.

3.  The electrosurgical generator according to claim 1 or 2, **characterized in that** the control module is configured to: in each sub-process, determine an ending impedance of current sub-process according to a minimum impedance and a bias impedance of the tissue in a previous sub-process; wherein the bias impedance is a fixed value, or a value that changes with sub-processes.

4.  The electrosurgical generator according to claim 3, **characterized in that**
    the control module is configured to end the current sub-process if a real-time impedance of the tissue is greater than the ending impedance of the current sub-process.

5.  The electrosurgical generator according to any one of claims 1 to 4,
    **characterized in that**:
    the control module is configured to end the current sub-process if a duration of outputting energy to the tissue according to the at least one control parameter of the current sub-process is greater than a preset timeout period.

6.  The electrosurgical generator according to any one of claims 1 to 5,
    **characterized in that**
    the control module is configured to end the current sub-process if a duration of the current sub-process is greater than a preset sub-process maximum duration.

7. The electrosurgical generator according to any one of claims 1 to 6, **characterized in that**:
the control module is further configured to: in each sub-process, determine an ending impedance at tissue closure according to a minimum impedance of the tissue in a previous sub-process, a minimum impedance of the tissue in all sub-processes before current sub-process, and an initial impedance of the tissue.

8. The electrosurgical generator according to claim 7, **characterized in that**
the control module is further configured to: before outputting energy to the tissue according to the at least one control parameter of current sub-process, determine whether tissue closure is completed based on a real-time impedance of the tissue and the ending impedance at tissue closure, wherein it is determined that tissue closure is completed when a real-time impedance of the tissue is greater than the ending impedance at tissue closure.

9. The electrosurgical generator according to any one of claims 1 to 8, **characterized in that**

the control module is further configured to: in determination of ending the current sub-process, determine whether tissue closure operation has timed out; and
proceed to the next sub-process when it is determined that the tissue closure operation is not timed out.

10. The electrosurgical generator according to any one of claims 1 to 9, **characterized in that**
the control module is further configured to:

obtain an initial impedance and initial phase of the tissue, where the initial phase of the tissue is an initial value of a phase difference between the voltage and current output to the tissue by the two electrodes of the cutter; and
determine whether the tissue is effectively clamped by the two electrodes of the cutter based on the initial impedance and the initial phase.

11. The electrosurgical generator according to claim 10, **characterized in that**
the control module is further configured to:

according to the initial phase, look up a table to obtain an impedance range corresponding to the initial impedance;
determine whether the initial impedance is within its corresponding impedance range; if yes, it is determined that the tissue is effectively clamped by the two electrodes of the cutter; if not, it is determine that the tissue is not effectively clamped by the two electrodes of the cutter.

12. The electrosurgical generator according to any one of claims 1 to 11, **characterized in that**
the control module is further configured to:

calculate an effective voltage value and an effective current value of the output energy based on the sampling signals, and calculate a reference impedance of the tissue according to the effective voltage value and effective current value;
calculate a voltage peak value and a current peak value for a base frequency of the output energy, as well as a voltage peak value and a current peak value for the second harmonic based on the sampling signals using a discrete Fourier transform algorithm;
calculate a base frequency impedance according to the voltage peak value and current peak value of the base frequency, and calculate a second harmonic impedance according to the voltage peak value and current peak value of the second harmonic;
determine a weight coefficient of the base frequency impedance based on a ratio of the reference impedance to the base frequency impedance, and determine a weight coefficient of the second harmonic impedance based on a ratio of the reference impedance to the second harmonic impedance; and
weighted average the base frequency impedance and the second harmonic impedance based on the weight coefficient of the base frequency impedance and the weight coefficient of the second harmonic impedance, to obtain a real-time impedance of the tissue.

13. The electrosurgical generator according to claim 12, **characterized in that**
the control module is further configured to:

calculate a reference phase of the output energy based on a voltage zero-crossing time point and a current zero-crossing time point in the sampling signals;

calculate a voltage phase and a current phase of the base frequency of the output energy, as well as a voltage phase and a current phase of the second harmonic of the output energy, using a discrete Fourier transform algorithm based on the sampling signals;

calculate a base frequency phase based on the voltage phase and current phase of the base frequency, and calculate a second harmonic phase based on the voltage phase and current phase of the second harmonic;

determine a weight coefficient of the base frequency phase based on a ratio of the reference phase to the base frequency phase, and determine a weight coefficient of the second harmonic phase based on a ratio of the reference phase to the second harmonic phase; and

weighted average the base frequency phase and the second harmonic phase based on the weight coefficient of the base frequency phase and the weight coefficient of the second harmonic phase, to obtain a real-time phase of the tissue.

14. An electrosurgical system, comprising:

the electrosurgical generator according to any one of claims 1 to 13 ; and

a cutter coupled to the electrosurgical generator, wherein the cutter comprises two electrodes for clamping tissue;

wherein energy is output to the tissue by the electrosurgical generator via the two electrodes of the cutter.

15. A control method applied to the electrosurgical generator according to any one of claims 1 to 13 or the electrosurgical system according to claim 14; wherein the method comprises:

executing a plurality of sub-processes sequentially after it is determined that the tissue has been effectively clamped by the two electrodes of the cutter;

during each sub-process,

determining at least one control parameter and at least one ending parameter of current sub-process based on at least one impedance parameter and at least one time parameter of the tissue; and

outputting energy to the tissue based on the at least one control parameter of the current sub-process, and determining whether the current sub-process should be ended based on the at least one ending parameter of the current sub-process.

**FIG.1**

**FIG.2**

FIG.3

**FIG.4**

S501

starting the tissue closure process

S502

obtaining the initial impedance and
initial phase of the tissue

S503

No ← determining
whether the tissue has been
effectively clamped by the
two electrodes of the
cutter

Yes

S504

loading algorithm data

S505

proceeding to the next sub-process
of the tissue closure process

S506

state determination stage, in which at least
one control parameter and at least one
ending parameter of the current
sub-process are determined based on at
least one impedance parameter of the
tissue and at least one time parameter

S507

determining
whether tissue closure is
completed → Yes

No

S508

tissue fusion stage, controlling the power
output module to output energy to the
tissue according to at least one control
parameter of the current sub-process,
and it is determined whether the current
sub-process should be ended based at
least one ending parameter of the
current sub-process

S509

prompting the user to
releasethe cutter and
re-clamp the tissue

S510

stopping energy output and
indicating that the tissue
closure is successful

**FIG.5**

S601

starting the tissue fusion stage

S602

outputting energy to the tissue
according to the power control curve

S603

No

$Z > Z_{localend}$,
or the tissue fusion stage has timed out,
or the sub-process has timed out

Yes

S604

Yes

determining
whether the tissue closure operation has
timed out

No

S605

indicating tissue
closure failure

S606

proceeding to
the next sub-process

**FIG.6**

**FIG.7**

**FIG.8**

# INTERNATIONAL SEARCH REPORT

| | International application No. |
|---|---|
| | **PCT/CN2022/128236** |

## A. CLASSIFICATION OF SUBJECT MATTER

A61B 18/14(2006.01)i; G06F 17/14(2006.01)i; G16H 40/63(2018.01)i

According to International Patent Classification (IPC) or to both national classification and IPC

## B. FIELDS SEARCHED

Minimum documentation searched (classification system followed by classification symbols)

A61B; G06F; G16H

Documentation searched other than minimum documentation to the extent that such documents are included in the fields searched

Electronic data base consulted during the international search (name of data base and, where practicable, search terms used)

CNTXT; ENTXTC; VEN; ENTXT; OETXT; CNKI; IEEE: 流程, 步骤, 过程, 周期, 阶段, 时间段, 时段, 阻抗, 初始, 最小, 相位, 时间, 偏置, 偏移, 结束, 基频, 谐波, 傅里叶变换, 当前, 实时, period, process, impedance, initial, minimum, phase, time, cycle, fourier transform, +procedure, circle, biasing, offset

## C. DOCUMENTS CONSIDERED TO BE RELEVANT

| Category* | Citation of document, with indication, where appropriate, of the relevant passages | Relevant to claim No. |
|---|---|---|
| PX | CN 114831725 A (INNOLCON MEDICAL TECHNOLOGY (SUZHOU) CO., LTD.) 02 August 2022 (2022-08-02) claims 1-14 | 1-14 |
| PX | CN 114886552 A (INNOLCON MEDICAL TECHNOLOGY (SUZHOU) CO., LTD.) 12 August 2022 (2022-08-12) description, paragraphs [0006]-[0213], figures 1-9 | 1-14 |
| PX | CN 115024814 A (INNOLCON MEDICAL TECHNOLOGY (SUZHOU) CO., LTD.) 09 September 2022 (2022-09-09) description, paragraphs [0007]-[0152], figures 1-9 | 1-14 |
| X | US 2012283731 A1 (UNGER JEFFREY R et al.) 08 November 2012 (2012-11-08) description paragraphs [0010]-[0087], figures 1-8 | 1-11, 14 |
| Y | US 2012283731 A1 (UNGER JEFFREY R et al.) 08 November 2012 (2012-11-08) description paragraphs [0010]-[0087], figures 1-8 | 12-14 |
| Y | US 2019201047 A1 (ETHICON LLC.) 04 July 2019 (2019-07-04) description, paragraphs [0533]-[0538] | 12-14 |

☑ Further documents are listed in the continuation of Box C.     ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **06 January 2023** | **16 January 2023** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2022/128236** |

## C. DOCUMENTS CONSIDERED TO BE RELEVANT

| Category* | Citation of document, with indication, where appropriate, of the relevant passages | Relevant to claim No. |
|---|---|---|
| A | CN 113811255 A (GYRUS ACMI, INC. (D.B.A. OLYMPUS SURGICAL TECHNOLOGIES AMERICA)) 17 December 2021 (2021-12-17) entire document | 1-14 |
| A | CN 109259849 A (CHONGQING JINSHAN SCIENCE & TECHNOLOGY (GROUP) CO., LTD.) 25 January 2019 (2019-01-25) entire document | 1-14 |

Form PCT/ISA/210 (second sheet) (January 2015)

**INTERNATIONAL SEARCH REPORT**

| International application No. |
|---|
| **PCT/CN2022/128236** |

| Box No. II    Observations where certain claims were found unsearchable (Continuation of item 2 of first sheet) |
|---|

This international search report has not been established in respect of certain claims under Article 17(2)(a) for the following reasons:

1. ☑ Claims Nos.: **15**
   because they relate to subject matter not required to be searched by this Authority, namely:

   [1]  Claim 15 sets forth a control method, the method comprising: after determining that two electrodes of a cutter effectively clamp tissue, sequentially performing a plurality of sub-processes, and outputting energy to the tissue by means of the two electrodes to achieve a closure process of the tissue, which falls within methods for treatment of the human or animal body by surgery or therapy, and therefore falls within subject matter for which no search is required by the International Searching Authority (PCT Rule 39.1(iv)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

INTERNATIONAL SEARCH REPORT
Information on patent family members

International application No.

**PCT/CN2022/128236**

| Patent document cited in search report | | | Publication date (day/month/year) | Patent family member(s) | | | Publication date (day/month/year) |
|---|---|---|---|---|---|---|---|
| CN | 114831725 | A | 02 August 2022 | None | | | |
| CN | 114886552 | A | 12 August 2022 | None | | | |
| CN | 115024814 | A | 09 September 2022 | None | | | |
| US | 2012283731 | A1 | 08 November 2012 | CA | 2574690 | A1 | 24 July 2007 |
| | | | | EP | 1810630 | A1 | 25 July 2007 |
| | | | | US | 2007173803 | A1 | 26 July 2007 |
| | | | | US | 2007173804 | A1 | 26 July 2007 |
| | | | | AU | 2007200268 | A1 | 09 August 2007 |
| | | | | AU | 2007200299 | A1 | 09 August 2007 |
| | | | | JP | 2007195973 | A | 09 August 2007 |
| | | | | US | 2009157071 | A1 | 18 June 2009 |
| | | | | US | 2009157072 | A1 | 18 June 2009 |
| | | | | US | 2009157075 | A1 | 18 June 2009 |
| | | | | US | 2010042093 | A9 | 18 February 2010 |
| | | | | CA | 2693934 | A1 | 23 August 2010 |
| | | | | AU | 2010200662 | A1 | 09 September 2010 |
| | | | | US | 7972328 | B2 | 05 July 2011 |
| | | | | US | 8147485 | B2 | 03 April 2012 |
| | | | | US | 8216223 | B2 | 10 July 2012 |
| | | | | JP | 2012161630 | A | 30 August 2012 |
| | | | | JP | 2012196458 | A | 18 October 2012 |
| | | | | AU | 2007200299 | B2 | 15 November 2012 |
| | | | | JP | 5085143 | B2 | 28 November 2012 |
| | | | | AU | 2007200268 | B2 | 04 April 2013 |
| | | | | AU | 2010200662 | B2 | 28 November 2013 |
| | | | | US | 8685016 | B2 | 01 April 2014 |
| | | | | US | 9186200 | B2 | 17 November 2015 |
| | | | | US | 2016045248 | A1 | 18 February 2016 |
| | | | | EP | 1810630 | B1 | 07 September 2016 |
| | | | | CA | 2693934 | C | 28 August 2018 |
| US | 2019201047 | A1 | 04 July 2019 | None | | | |
| CN | 113811255 | A | 17 December 2021 | WO | 2020227519 | A1 | 12 November 2020 |
| | | | | US | 2020352618 | A1 | 12 November 2020 |
| | | | | US | 2020352624 | A1 | 12 November 2020 |
| | | | | US | 2020352625 | A1 | 12 November 2020 |
| | | | | US | 2020352626 | A1 | 12 November 2020 |
| | | | | US | 2020352627 | A1 | 12 November 2020 |
| | | | | US | 2020352628 | A1 | 12 November 2020 |
| | | | | US | 2020352629 | A1 | 12 November 2020 |
| | | | | US | 2020352630 | A1 | 12 November 2020 |
| | | | | US | 2020352631 | A1 | 12 November 2020 |
| | | | | US | 2020352632 | A1 | 12 November 2020 |
| | | | | US | 2020352635 | A1 | 12 November 2020 |
| | | | | US | 2020352636 | A1 | 12 November 2020 |
| | | | | US | 2020352637 | A1 | 12 November 2020 |
| | | | | US | 2020352638 | A1 | 12 November 2020 |
| | | | | US | 2020352639 | A1 | 12 November 2020 |
| | | | | AU | 2020267650 | A1 | 09 December 2021 |
| | | | | AU | 2020267650 | A8 | 23 December 2021 |

Form PCT/ISA/210 (patent family annex) (January 2015)

**INTERNATIONAL SEARCH REPORT**
Information on patent family members

International application No.

**PCT/CN2022/128236**

| Patent document cited in search report | | | Publication date (day/month/year) | Patent family member(s) | | | Publication date (day/month/year) |
|---|---|---|---|---|---|---|---|
| | | | | KR | 20220007884 | A | 19 January 2022 |
| | | | | EP | 3965676 | A1 | 16 March 2022 |
| | | | | JP | 2022531724 | A | 08 July 2022 |
| CN | 109259849 | A | 25 January 2019 | CN | 109259849 | B | 05 January 2021 |

Form PCT/ISA/210 (patent family annex) (January 2015)